# EUROPEAN PATENT APPLICATION

(11) **EP 1 138 772 A1**
(43) Date of publication of application: **04.10.2001**
(21) Application number: 99961284.9
(22) Date of filing: 09.12.1999
(51) Int. Cl.: C12N 15/62, C12N 15/12, C12N 15/18, C12P 21/02, C12N 1/21, C07K 19/00, C07K 14/47, C07K 14/50, C07K 1/107, C07K 1/12

(54) **PROCESS FOR PRODUCING APELIN**

(30) Priority: 11.12.1998 JP 35238998; 04.10.1999 WO PCT/JP99/05456
(71) Applicant: Takeda Chemical Industries, Ltd., Osaka-shi, Osaka 541-0045 (JP)
(72) Inventor: SUENAGA, Masato, Hyogo 663-8105 (JP); ITO, Takashi, Kobe-shi Hyogo 658-0032 (JP); NISHIMURA, Osamu, Ibaraki 305-0812 (JP)
(74) Representative: Best, Michael, Dr.
(86) International application number: JP9906903
(87) International publication number: WO0036122

(57) **Abstract**

The present invention provides a production method advantageous in producing Apelin on an industrial scale in large amounts.

The present invention relates to a method for producing Apelin or a salt thereof, comprising subjecting a fused protein or peptide consisting of a protein or peptide having N-terminal cysteine with Apelin optionally having an optionally oxidized methionine residue at the N-terminus thereof joined to the N-terminus of the protein or peptide to a cleavage reaction for the peptide bond on the amino group side of the cysteine residue.

Using the production method of the present invention, it is possible to produce on an industrial scale in large amounts Apelin that can be used as a therapeutic/preventive agent for such diseases as various forms of dementia, including senile dementia and cerebrovascular dementia, depression, hyperkinetic (minimal brain damage) syndrome, disturbance of consciousness, anxiety disorder, schizophrenia, phobia, impairments of growth hormone secretion, hyperphagia, polyphagia, hypercholesterolemia, hyperglyceridemia, hyperlipidemia, hyperprolactinemia, hypoglycemia, hypopituitarism, pituitary dwarfism, diabetes mellitus, cancers, pancreatitis, renal diseases, Turner's syndrome, neurosis, rheumatoid arthritis, spinal injury, transient cerebral ischemic attack, amyotrophic lateral sclerosis, acute myocardial infarction, spinocerebellar deformation, bone fractures, wounds, atopic dermatitis, osteoporosis, asthma, epilepsy, infertility, arteriosclerosis, pulmonary emphysema, pulmonary edema, and milk secretion insufficiency, and can also be used as a hypnotic sedative, a postoperative nutritional status improving agent, a hypertensive, a hypotensive, a preventive or therapeutic drug for HIV infection, AIDS, etc., and the like.

## Description

### FIELD OF THE INVENTION

The present invention relates to a method for producing Apelin or a salt thereof, comprising producing a fused protein or a polypeptide, and subsequently subjecting said fused protein or peptide to a cleavage reaction for peptide bonds. The present invention also relates to a method for efficiently removing an optionally oxidized methionine residue at the N-terminus or a diketone derivative of said methionine residue from Apelin having the optionally oxidized methionine residue at the N-terminus or a salt thereof.

### BACKGROUND ART

In producing a peptide using gene recombination technology, it is often expressed in the form of a fused protein, since peptides are likely to be decomposed in cells. For cleaving out a desired peptide from a fused protein, a method involving chemical cleavage using cyanogen bromide [Itakura et al., Science, 198, 1056 (1977)] and a method involving enzymatic cleavage using factor Xa [Nagai et al., Methods in Enzymology, 153, 46 (1987)] are available.

Furthermore, among known methods for cleaving peptide bonds in a protein is cleavage of acylcysteine bonds using 2-nitro-5-thiocyanobenzoic acid ("Seikagaku Jikken Koza" 1, Tanpakushitsu no Kagaku II, edited by the Japanese Biochemical Society, published by Tokyo Kagaku Dojin, pp. 247-250, 1976). However, no disclosure is given regarding cleaving out a desired peptide from a protein.

It is known that when a protein is biosynthesized in a cell, its N-terminus starts with methionine, which corresponds to the mRNA initiation codon AUG. However, because of removal by subsequent processing, this methionine is usually no longer present in the resulting mature protein molecule.

Thanks to advances in gene recombination technology, it has become possible to produce useful proteins using microorganisms and animal cells, e.g., *Escherichia coli,* and some proteins produced by this approach retain such methionine. For example, the methionine addition rate is nearly 100% in human growth hormone expressed in *Escherichia coli* [Nature, 293, 408 (1981)], and the rate is 50% in interferon-α [Journal of Interferon Research, 1, 381 (1981)]; in non-glycosylated human interleukin-2, a molecular species (Met-rIL-2) with additional methionine added to the amino-terminus (having a methionine residue at the N-terminus) is found, as well as a molecular species (rIL-2) starting with alanine as natural human interleukin-2.

Regarding methods for chemically removing N-terminal amino acids, Dixon reported that when DL-alanylglycine was reacted with glyoxylic acid, pyridine, and copper acetate, an amino group transamination reaction took place to produce pyruvoylglycine [Biochemistry Journal, 92, 661 (1964)], and that when the resulting compound was reacted with thiosemicarbazide, cleavage of the amide bond occurs to produce glycine [Biochemistry Journal, 90, 2C (1964)]. The same author also reported that when this reaction was applied to cytochrome c-551 (Pseudomonas cytochrome c-551), the N-terminal glutamic acid was removed [Biochemistry Journal, 94, 463 (1965)].

As far as the prior art is concerned, the use of cyanogen bromide in cleaving out a desired peptide from a fused protein is not applicable to the production of a methionine-containing peptide, and poses many problems, including recovery rate at the time of cleavage.

Accordingly, there is a demand for a method for efficiently cleaving out a desired peptide from a fused protein or a polypeptide.

Furthermore, even within the same protein, a molecular species with methionine added to the N-terminus and another molecular species without it can have mutually different properties in terms of higher structure, biological activity, and stability of protein, and it is hypothesized that the addition of methionine to the N-terminus can result in increased antigenicity. From the viewpoint of industrial application, it is believed significant to establish a method for removing N-terminal methionine corresponding to the initiation codon.

To resolve this problem, there was proposed a method for removing methionine by cyanogen bromide (BrCN) decomposition [Science, 198, 1056 (1977)]. In this case, however, a methionine residue must not be present in the desired mature protein, and satisfactory results are never obtained by this method, which involves a stringent chemical reaction on the protein.

There is no chemical method known to enable the selective and efficient removal of the N-terminal methionine residue from a peptide or protein having a methionine residue at the N-terminus thereof, irrespective of the kind of peptide or protein, except for the method described in Japanese Patent Unexamined Publication No. 72489/1998 (EP-A-812856). This is attributable to difficulty in finding out a chemical reaction capable of removing the N-terminal methionine residue under mild conditions without denaturing the final product peptide or protein. In removing undesirably added methionine at the N-terminus from a protein of relatively high molecular weight produced using gene engineering technology, especially a protein intended for pharmaceutical use, the reaction must usually be advanced in a weakly acidic to weakly alkaline aqueous solution without heating because the protein activity must not decrease after methionine removal; there are many limitations in selecting chemical reaction conditions so that no favorable reaction conditions have been found.

### DISCLOSURE OF THE INVENTION

The present inventors conducted extensive investigation in an attempt to develop a method for efficiently producing the novel bioactive peptide Apelin [Biochem. Bibphys. Res. Commun., 251, 471-476 (1998)] or a salt thereof, and found that Apelin can be produced efficiently by producing a fused protein or polypeptide consisting of a protein or polypeptide having N-terminal cysteine with Apelin joined to the N-terminus thereof, and subsequently subjecting said fused protein or peptide to a reaction that cleaves peptide bonds.

Furthermore, the present inventors conducted extensive investigation aiming at providing a production method for Apelin having a naturally occurring amino acid sequence by selectively cleaving the N-terminal methionine residue in Apelin produced using gene engineering technology, and found that by reacting Apelin having an optionally oxidized methionine residue at the N-terminus thereof, represented by formula (I), with, for example, glyoxylic acid, which is an α-diketone, copper sulfate, which is capable of releasing a transition metal ion, and pyridine, which is a base (e.g., amine), to carry out an amino group transamination reaction, reacting the resulting Apelin having a diketone derivative of said methionine residue with 3,4-diaminobenzoic acid, which is a base (e.g., diamine), to carry out a hydrolysis reaction, as shown in scheme 1 below, the diketone derivative of the methionine residue can be removed unexpectedly efficiently from said Apelin having the diketone derivative of the methionine residue. Specifically, the present inventors discovered a method enabling the obtainment of Apelin not having an optionally oxidized methionine residue at the N-terminus thereof at an unexpectedly high recovery rate, without reducing its activity, by removing the optionally oxidized methionine residue at the N-terminus from Apelin having a methionine residue. The inventors conducted further study based on this finding, and developed the present invention.

### (Scheme 1)

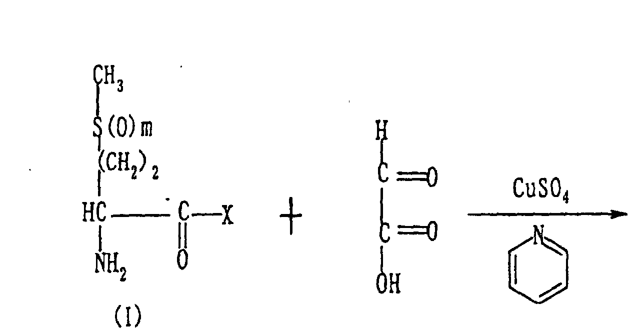

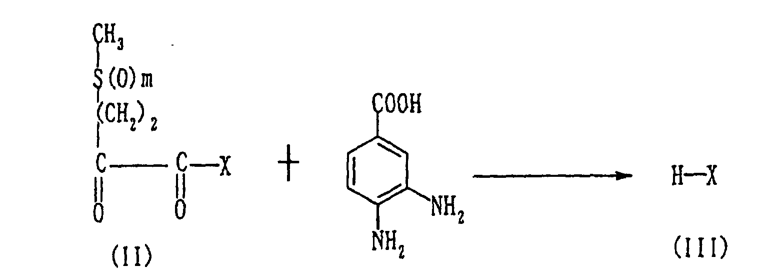

[In formula (I), m represents an integer from 0 to 2; X represents a peptide chain of Apelin.]

In the present specification, the following designations are also used with regard to scheme 1 above:
(1) a compound represented by general formula (I) is also referred to as "Apelin having an optionally oxidized methionine residue at the N-terminus thereof or a salt thereof" or "Apelin having a methionine residue or a salt thereof";
(2) a partial structure represented by the formula: 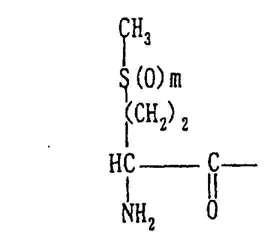 [m has the same definition as that given above] in general formula (I) as "optionally oxidized methionine residue," 'methionine residue" or "methionine";
(3) a compound represented by general formula (II) as "Apelin having a diketone derivative of an optionally oxidized methionine residue at the N-terminus thereof or a salt thereof" or "Apelin having a diketone derivative of a methionine residue or a salt thereof";
(4) a partial structure represented by the formula: 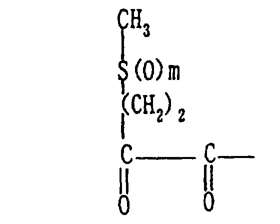 [m has the same definition as that given above] in general formula (II) as 'diketone derivative of an optionally oxidized methionine residue" or 'diketone derivative of a methionine residue"; and
(5) a compound typically represented by general formula (III) as "Apelin not having an optionally oxidized methionine residue at the N-terminus thereof or a salt thereof" or "Apelin not having a diketone derivative of an optionally oxidized methionine residue at the N-terminus thereof or a salt thereof."

The present invention relates to:
(1) a method for producing Apelin or a salt thereof, comprising subjecting a fused protein or peptide consisting of a protein or peptide having N-terminal cysteine with Apelin optionally having an optionally oxidized methionine residue at the N-terminus thereof joined to the N-terminus of the protein or peptide, to a cleavage reaction for the peptide bond on the amino group side of the cysteine residue,
(2) a method for producing Apelin optionally having a methionine residue at the N-terminus thereof or a salt thereof, comprising culturing a transformant harboring a vector having a gene that encodes a fused protein or peptide consisting of a protein or peptide having N-terminal cysteine with Apelin optionally having a methionine residue at the N-terminus thereof joined to the N-terminus of the protein or peptide, to allow it to express the fused protein or peptide, and subjecting the fused protein or peptide thus expressed to a cleavage reaction for the peptide bond on the amino group side of the cysteine residue,
(3) the production method of (1) or (2) above, wherein said cleavage reaction involves an S-cyanylation reaction and a subsequent hydrolysis reaction,
(4) the production method of (1) or (2) above, wherein said Apelin is a polypeptide containing the amino acid sequence shown by SEQ ID NO: 1,
(5) a fused protein or peptide consisting of a protein or peptide having N-terminal cysteine with Apelin optionally having a methionine residue at the N-terminus thereof joined to the N-terminus of the protein or peptide,
(6) a vector having a gene that encodes the fused protein or peptide of (5) above,
(7) a transformant containing the vector of (6) above,
(8) a method for removing an optionally oxidized methionine residue, comprising reacting Apelin having the optionally oxidized methionine residue at the N-terminus thereof or a salt thereof with an α-diketone, and subsequently conducting hydrolysis,
(9) the method of (8) above, wherein said Apelin having an optionally oxidized methionine residue at the N-terminus thereof is produced using gene engineering technology,
(10) the method of (8) above, comprising reacting with an α-diketone in the presence of a transition metal ion,
(11) the method of (8) above, comprising reacting with an α-diketone in the presence of a base,
(12) the method of (8) above, comprising reacting with an α-diketone in the presence of a transition metal ion and a base,
(13) the method of (8) above, wherein said α-diketone is glyoxylic acid or a salt thereof,
(14) the method of (10) above, wherein said transition metal ion is a copper ion,
(15) the method of (11) above, wherein said base is pyridine,
(16) the method of (8) above, comprising conducting hydrolysis using a base,
(17) the method of (16) above, wherein said base is an amine,
(18) the method of (16) above, wherein said base is a diamine or a thio or selenosemicarbazide,
(19) the method of (18) above, wherein said diamine is o-phenylenediamine or 3,4-diaminobenzoic acid,
(20) a method for producing Apelin or a salt thereof, comprising reacting Apelin produced using gene engineering technology and having methionine added to the N-terminus thereof or a salt thereof with glyoxylic acid or a salt thereof in the presence of copper sulfate and pyridine, and subsequently reacting with o-phenylenediamine or 3,4-diaminobenzoic acid,
(21) a compound represented by the formula CH₃-S(O)ₘ-(CH₂)₂-CO-CO-X [m represents an integer from 0 to 2; X represents a peptide chain of Apelin] or a salt thereof,
(22) a method for producing Apelin or a salt thereof, comprising hydrolyzing the compound of (21) above, and
(23) a method for producing Apelin or a salt thereof, comprising subjecting a fused protein or peptide consisting of a protein or peptide having N-terminal cysteine with Apelin having a methionine residue at the N-terminus thereof joined to the N-terminus of the protein or peptide, to a cleavage reaction for the peptide bond on the amino group side of the cysteine residue, to yield Apelin having an optionally oxidized methionine residue at the N-terminus thereof or a salt thereof, further reacting said Apelin having an optionally oxidized methionine residue at the N-terminus thereof or a salt thereof with an α-diketone, and subsequently conducting hydrolysis.

### BRIEF DESCRIPTION OF THE DRAWINGS

Fig. 1 shows the mechanism of reaction in the reaction process of the present invention.
Fig. 2 shows the DNA fragment used in Example 1.
Fig. 3 shows a schematic diagram for the production of the double-stranded human Apelin-36 obtained in Example 1.
Fig. 4 shows a construction scheme for the plasmid pTB960-13 obtained in Example 2.

### BEST MODE OF EMBODIMENT OF THE INVENTION

Forms of Apelin useful for the method of the present invention include, for example, human Apelin-36 (polypeptide of the amino acid sequence shown by SEQ ID NO: 1), described in Biochem. Biophys. Res. Commun., 251, 471-476 (1998), Apelin-13 (polypeptide of the amino acid sequence shown by the 24th through 36th amino acids in SEQ ID NO: 1), and a peptide resulting from pyroglutamination of the N-terminal amino acid (Gln) of Apelin-13. Any peptide can serve for the method of the present invention, as long as it possesses ligand activity for APJ (O'Dowd. B.F., et al., Gene, 436, 355-359, 1993); specifically, there may be mentioned, for example, the "polypeptide capable of binding to a receptor protein containing the same or substantially the same amino acid sequence as the amino acid sequence shown by SEQ ID NO: 3" described in WO 99/33976 (Japanese Patent Application No. 364656/1998).

The scope of the Apelin of the present invention includes those forms wherein Gln is pyroglutaminated upon cleavage on the N-terminal side in the living body.

In the present specification, peptides are described as having an N-terminus (amino terminus) at the left end and a C-terminus (carboxyl terminus) at the right end, as is common practice of describing peptides. The C-terminus of the peptide shown by SEQ ID NO: 1 may be a carboxyl group (-COOH), a carboxylate (-COO⁻), an amide (-CONH₂), an alkylamide (-CONHR) or an ester (-COOR). The R for an ester or an alkylamide is exemplified by a C₁₋₆ alkyl group such as methyl, ethyl, n-propyl, isopropyl and n-butyl; a C₃₋₈ cycloalkyl group such as cyclopentyl and cyclohexyl; a C₆₋₁₂ aryl group such as phenyl and α-naphthyl; phenyl-C₁₋₂ alkyl such as benzyl, phenethyl and benzhydryl; or a C₇₋₁₄ aralkyl group such as α-naphthyl-C₁₋₂ alkyl such as α-naphthylmethyl; as well as pivaloyloxymethyl esters, which are in common use for oral administration.

The salt of the Apelin of the present invention is a salt with a physiologically acceptable base (e.g., alkali metal) and is preferably a physiologically acceptable acid adduct salt. As such salts, there may be used, for example, salts with inorganic acids (e.g., hydrochloric acid, phosphoric acid, hydrobromic acid, sulfuric acid) and salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The protein or peptide used for the method of the present invention, which has N-terminal cysteine, is not subject to limitation. A protein or peptide not having N-terminal cysteine can also serve for the present invention, as long as it is made to have N-terminal cysteine using a commonly known method.

Said protein or peptide, which has N-terminal cysteine, preferably has a molecular weight of 100 to 100,000, more preferably 300 to 50,000. The protein or peptide having N-terminal cysteine preferably has 1 to 1,000 amino acids, more preferably 3 to 500 amino acids.

Said protein or peptide is exemplified by various growth factors such as interferons, interleukins, fibroblast growth factors (aFGF, bFGF, etc.); enzyme proteins such as (pro)urokinases, lymphotoxins, tumor necrosis factor (TNF), β-galactosidase; reserve proteins; streptoavidin; protein A, protein G, tissue plasminogen activator (TPA), muteins thereof or portions (fragments) thereof; having N-terminal cysteine. Preference is given to fibroblast growth factors (aFGF, bFGF, etc.) or muteins thereof or portions (fragments) thereof (e.g., bFGF CS23 muteins).

As a bFGF CS23 mutein, there may be mentioned, for example, a protein containing the amino acid sequence shown by Pro-Ala-Leu-Pro-Glu-Asp-Gly-Gly-Ser-Gly-Ala-Phe-Pro-Pro-Gly-His-Phe-Lys-Asp-Pro-Lys-Arg-Leu-Tyr-Cys-Lys-Asn-Gly-Gly-Phe-Phe-Leu-Arg-Ile-His-Pro-Asp-Gly-Arg-Val-Asp-Gly-Val-Arg-Glu-Lys-Ser-Asp-Pro-His-Ile-Lys-Leu-Gln-Leu-Gln-Ala-Glu-Glu-Arg-Gly-Val-Val-Ser-Ile-Lys-Gly-Val-Ser-Ala-Asn-Arg-Tyr-Leu-Ala-Met-Lys-Glu-Asp-Gly-Arg-Leu-Leu-Ala-Ser-Lys-Ser-Val-Thr-Asp-Glu-Cys-Phe-Phe-Phe-Glu-Arg-Leu-Glu-Ser-Asn-Asn-Tyr-Asn-Thr-Tyr-Arg-Ser-Arg-Lys-Tyr-Thr-Ser-Trp-Tyr-Val-Ala-Leu-Lys-Arg-Thr-Gly-Gln-Tyr-Lys-Leu-Gly-Ser-Lys-Thr-Gly-Pro-Gly-Gln-Lys-Ala-Ile-Leu-Phe-Leu-Pro-Met-Ser-Ala-Lys-Ser (SEQ ID NO: 3), and having a cysteine residue added to the N-terminus thereof.

The gene that encodes a fused protein (fused peptide included) useful for the method of the present invention may be constructed by (1) chemically synthesizing the entire base sequence, or (2) placing a cysteine-encoding base sequence on the N-terminus side of a protein-encoding base sequence, and further placing an Apelin-encoding base sequence on the N-terminus side. Alternatively, (3) when the purpose is to obtain a fragment of said peptide, said gene may be constructed by replacing an amino acid residue just after the desired fragment with cysteine using a technique such as site-directed mutagenesis.

Regarding the production method in case (1) above, the phosphoamidide method, the phosphoric acid triester method, the diester method, the hydrogen phosphonate method, or another commonly known method may be used to synthesize said base sequence at one time for a short sequence, or in portions to be ligated using T4 DNA ligase for a long sequence.

Regarding the production method in case (2) above, a gene that encodes the protein on the C-terminal side is obtained by cutting out from a chromosome using the appropriate restriction endonuclease, and joining the fragment to a vector, or by deriving a cDNA. The gene is then cleaved using a restriction endonuclease to have N-terminal cysteine, or a synthetic DNA is bound to the 5'-terminus of the gene for the whole protein or a portion thereof to modify the N-terminus to have cysteine. To the 5'-terminus, a gene that encodes the desired protein (which may be synthesized chemically or cloned from a living organism) is ligated.

As a specific example of the thus-obtained fused protein-encoding gene, there may be mentioned, for example, the DNA represented by the formula 
CTGGTGCAGCCCAGAGGGTCAAGGAATGGGCCAGGGCCCTGGCAGGGAGGTCGGAGGAA
ATTCCGCCGCCAGCGGCCCCGCCTCTCCCATAAGGACCCATGCCTTTC-TGC or
TGT-R (I)
 [R represents a base sequence consisting of

CCCGAGGATGGCGGCAGCGGCGCCTTCCCGCCCGGCCACTTCAAGGACCCCAAGCGGCT
GTACTGCAAAAACGGGGGCTTCTTCCTGCGCATCCACCCGACGGCCGAGTTGACGGGT
CCGGGAGAAGAGCGACCCTCACATCAAGCTACAACTTCAAGCAGAAGAGAGAGGAGTTG
TGTCTATCAAAGGAGTGAGCGCTAATCGTTACCTGGCTATGAAGGAAGATGGAAGATTA

CTAGCTTCTAAGTCTGTTACGGATGAGTGTTTCTTTTTGAACGATTGGAATCTAATAA
CTACAATACTTACCGGTCAAGGAAATACACCAGTTGGTATGTGGCACTGAAACGAACTG
GGCAGTATAAACTTGGATCCAAAACAGGACCTGGGCAGAAAGCTATACTTTTTCTTCCA
ATGTCTGCTAAGACTGC (fragment of hbFGF mutein CS23)].

Formula (I) above shows that a base sequence represented by R is bound, via a cysteine-encoding base sequence, to a DNA base sequence that encodes a polypeptide containing human Apelin-36 (SEQ ID NO: 2).

A DNA (plasmid) having ATG at the 5'-terminus thereof, a region that encodes said fused protein downstream thereof, and a translation termination codon adjoining said region, can be produced by processing a known cDNA of said protein produced by chemical synthesis or gene engineering technology, or a DNA of said protein of chromosomal origin.

The gene of the present invention, which encodes a fused protein or peptide, consisting of a protein or peptide having N-terminal cysteine and Apelin joined to the N-terminus of the protein or peptide, may be converted to a gene that encodes the desired mutein using a conventional DNA technology, e.g., site-directed mutagenesis technology.

Site-directed mutagenesis technology is obvious to those skilled in the art, and is described by Lather, R.F. and Lecoq, J.P. in Genetic Engineering, Academic Press Company (1983), pp. 31-50. Oligonucleotide-directed mutagenesis is described by Smith, M. and Gillam, S. in Genetic Engineering: Principles and Methods, Plenam Press Company (1981), Vol. 3, pp. 1-32.

Plasmids used as vectors for producing a plasmid having a DNA having a region that encodes said fused protein include, for example, *Escherichia coli*-derived plasmids, such as pBR322 [Gene, 2, 95 (1977)], pBR313 [Gene, 2, 75 (1977)], pBR324, pBR325 [Gene, 4, 124 (1978)], pBR327, pBR328 [Gene, 9, 287 (1980)], pBR329 [Gene, 17, 79 (1982)], pKY2289 [Gene, 3, 1 (1978)], pKY 2700 [Seikagaku, 52, 770 (1980)], pACYC177, pACYC184 [Journal of Bacteriology, 134, 1141 (1978)], pRK248, pRK646, pDF [Methods in Enzymology, 68, 268 (1979)], pUC18, and pUC19 [Yanish-Peron et al., Gene, 33, 103 (1985)]. There may also be mentioned λgt-series plasmids using bacteriophages, e.g., λ phage, such as λgt·λC [Proc. Natl. Acad. Sci. U.S.A., 71, 4579 (1974)], λgt·λB [Proc. Natl. Acad. Sci. U.S.A., 72, 3461 (1975)], and λDam [Gene, 1, 255 (1977)], the Sharon vector [Science, 196, 161 (1977); Journal of Virology, 29, 555 (1979)], and mp-series plasmids using fibrous phages, such as mp18 and mp19 [Yanish-Peron et al., Gene, 33, 103 (1985)].

The DNA described above preferably has a promoter upstream of ATG, which promoter may be any one, as long as it is appropriate to the host used to produce a transformant.

For example, the trp promoter, the lac promoter, the recA promoter, the λPL promoter, the lpp promoter, the T7 promoter, etc., for *Escherichia coli,* the SPO1 promoter, the SPO2 promoter, the penP promoter, etc., for *Bacillus subtilis,* the PHO5 promoter, the PGK promoter, the GAP promoter, the ADH promoter, etc., for *Saccharomyces cerevisiae*, and the SV40-derived promoter etc., for animal cells. An SD (Shine-and-Dalgarno) sequence may be inserted downstream of the promoter as necessary.

When a T7 promoter system is used, the T7 promoter may be any of the 17 kinds of promoters found on T7 DNA [J.L. Oakley et al., Proc. Natl. Acad. Sci. U.S.A., 74, 4266-4270 (1977)]; M.D. Rosa, Cell, 16, 815-825 (1979); N. Panayotatos et al., Nature, 280, 35 (1979); J.J. Dunn et al., J. Mol. Biol., 166, 477-535 (1983), with preference given to the φ10 promoter [A.H. Rosenberg et al., Gene, 56, 125-135 (1987)].

As transcription terminators, there may be used terminators serving in *Escherichia coli* systems, with preference given to the Tφ terminator [F.W. Studier et al., J. Mol. Biol., 189, 113-130 (1986)].

T7 RNA polymerase genes are exemplified by the T7 gene [F.W. Studier et al., J. Mol. Biol., 189, 113-130 (1986)].

The vector is preferably constructed by incorporating the T7 promoter and the T7 terminator into a vector described above. Such vectors include pET-1, pET-2, pET-3, pET-4, pET-5 [A.H. Rosenberg et al., Gene, 56, 125-135 (1987)], and pTB960-2 [EP-A-499990], with preference given to pTB960-2.

The transformant of the present invention can be produced by transforming a host with an expression plasmid obtained using the method described above, using a commonly known method [e.g., Cohen S.N. et al., Proc. Natl. Acad. Sci. U.S.A., 69, 2110 (1972)].

Microbial hosts to be transformed include, for example, bacteria of the genus *Escherichia,* bacteria of the genus *Bacillus,* yeasts, and animal cells.

Examples of such bacteria of the genus *Escherichia* include *Escherichia coli*, more specifically *Escherichia coli* K12DH1 [Proc. Natl. Acad. Sci. U.S.A., 60, 160 (1968)], JM-103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], N4830 [Cell, 25, 713 (1981)], K-12MM294 [Proc. Natl. Acad. Sci. U.S.A., 73, 4174 (1976)], and BL-21.

Such bacteria of the genus *Bacillus* include, for example, *Bacillus subtilis*, more specifically *Bacillus subtilis* MI114 [Gene, 24, 255 (1983)] and 207-21 [Journal of Biochemistry, 95, 87 (1984)].

Such yeasts include, for example, *Saccharomyces cerevisiae*, more specifically *Saccharomyces cerevisiae* AH22 [Proc. Natl. Acad. Sci. U.S.A., 75, 1929 (1978)], XSB52-23C [Proc. Natl. Acad. Sci. U.S.A., 77, 2173 (1980)], BH-641A (ATCC 28339), 20B-12 [Genetics, 85, 23 (1976)], and GM3C-2 [Proc. Natl. Acad. Sci. U.S.A., 78, 2258 (1981)].

Animal cells include, for example, simian COS-7 cells [Cell, 23, 175 (1981)], Vero [Japanese Journal of Clinical Medicine, 21, 1209 (1963)], Chinese hamster CHO cells [Journal of Experimental Medicine, 108, 945 (1985)], mouse L cells [Journal of National Cancer Institute, 4, 165 (1943)], human FL cells [Proceedings of the Society for Experimental Biology and Medicine, 94, 532 (1957)], and hamster C cells.

When a T7 promoter system is used, the host for the transformant may be any strain of *Escherichia coli* incorporating the T7 RNA polymerase gene (T7 gene 1) [F.W. Studier et al., J. Mol. Biol., 189, 113-130 (1986)], e.g., MM294, DH-1, C600, JM109, and BL21, or any strain of *Escherichia coli* incorporating the T7 RNA polymerase gene (T7 gene 1) along with another plasmid. Preferably, the MM294 strain and the BL21 strain are used wherein λ phage incorporating the T7 gene 1 is lysogenized. In this case, as the promoter for the T7 gene 1, the lac promoter, wherein expression is induced by isopropyl-1-thio-β-D-galactopyranoside (also referred to as IPTG) is used.

Transformation of a bacterium of the genus *Bacillus* as the host can be achieved using a commonly known method, such as that described in Molecular and General Genetics, 168, 111 (1979).

Transformation of a yeast as the host can be achieved using a commonly known method, such as that described in Proc. Natl. Acad. Sci. U.S.A., 75, 1929 (1978).

Transformation of an animal cell as the host can be achieved using a commonly known method, such as that described in Virology, 52, 456 (1973).

A fused protein can be produced by culturing a transformant described above in a medium, and collecting the fused protein produced.

The pH of the medium is desirably about 6 to 8.

As the medium for culturing a bacterium of the genus *Escherichia,* there may be preferably used, for example, an M9 medium containing glucose and casamino acid [Miller, Journal of Experiments in Molecular Genetics, 431-433, Cold Spring Harbor Laboratory, New York (1972)]. To increase promoter efficiency as necessary, a chemical, e.g., 3β-indolylacrylic acid or isopropyl-β-D-thiogalactopyranoside (IPTG), may be added to this medium.

When the host is a bacterium of the genus Escherichia, cultivation is normally carried out at about 15 to 43°C for about 3 to 24 hours, with aeration and stirring added as necessary.

When the host is a bacterium of the genus *Bacillus,* cultivation is normally carried out at about 30 to 40°C for about 6 to 24 hours, with aeration and stirring added as necessary.

As the medium for culturing a transformant whose host is a yeast, there may be mentioned, for example, Burkholder's minimal medium [Bostian, K.L. et al., Proc. Natl. Acad. Sci. U.S.A., 77, 4505 (1980)]. The pH of the medium is preferably adjusted to about 5 to 8. Cultivation is normally carried out at about 20 to 35°C for about 24 to 72 hours, with aeration and stirring added as necessary.

As the medium for culturing a transformant whose host is an animal cell, there may be mentioned, for example, an MEM medium containing about 0.2 to 20%, preferably about 5 to 20%, fetal bovine serum [Science, 122, 501 (1952)], DME medium [Virology, 8, 396 (1959)], RPMI 1640 medium [Journal of the American Medical Association, 199, 519 (1967)], and 199 medium [Proceedings of the Society for the Biological Medicine, 73, 1 (1950)]. The pH is preferably about 6 to 8. Cultivation is normally carried out at about 30 to 40°C for about 15 to 60 hours, with aeration and stirring added as necessary.

A fused protein can be produced by culturing a transformant described above, and collecting the fused protein produced and accumulated in the culture.

As the medium, there may be mentioned, for example, an M9 medium containing glucose and casamino acid [Miller, Journal of Experiments in Molecular Genetics, 431-433, Cold Spring Harbor Laboratory, New York, 1972], 2 × YT medium [Messing, Methods in Enzymology, 101, 20 (1983)], and LB medium.

Cultivation is normally carried out at about 15 to 43°C for about 3 to 24 hours, with aeration and stirring added as necessary.

When a recombinant having both the λcIts repressor and an expression vector containing the λP_{L}-promoter is used, cultivation is normally carried out at about 15 to 36°C, preferably about 30 to 36°C, and the λcIts repressor is preferably inactivated at about 37 to 42°C. To increase the efficiency of the recA promoter, i.e., to reduce recA gene expression-suppressing function, a chemical, such as mitomycin C or nalidixic acid, may be added, ultraviolet light may be irradiated, or the pH of the culture broth may be shifted to an alkaline value.

When a T7 promoter system is used, the T7 promoter is specifically caused to function by the resulting T7 phage RNA polymerase I, by (1) adding IPTG or the like in cases of expression of the T7 gene (RNA polymerase gene) joined downstream of the lac promoter, or (2) increasing the culturing temperature in cases of expression of the T7 gene (RNA polymerase gene) joined downstream of the λP_{L} promoter, or the like.

After cultivation, cells are harvested using a commonly known method and suspended in a buffer solution, for example, after which they are disrupted by means of, for example, protein denaturant treatment, sonication, enzyme treatment such as with lysozyme, glass bead treatment, French press treatment, lyophilization, or the like, and subjected to a commonly known method, such as centrifugation, to yield a supernatant.

A fused protein can be isolated from the supernatant obtained above, using an ordinary method of protein purification. For example, this isolation can be achieved by appropriately combining gel filtration, ion exchange chromatography, adsorption chromatography, high performance liquid chromatography, affinity chromatography, hydrophobicity chromatography, electrophoresis, etc. The thus-obtained fused protein may have methionine of translation initiation codon origin added to the N-terminus thereof. Said fused protein may be forwarded to the next reaction process without purification or with partial purification.

Next, the fused protein or peptide thus obtained is subjected to a cleavage reaction for the peptide bond on the amino group side of the cysteine residue. Said cleavage reaction is exemplified by an S-cyanylation reaction and subsequent hydrolysis reaction. When an amide of Apelin or a salt thereof is obtained as the final product, said cleavage reaction is exemplified by an S-cyanylation reaction and subsequent ammonolysis. Said S-cyanylation reaction is carried out by reacting the starting compound with an S-cyanylation reagent.

S-cyanylation reagents include, for example, 2-nitro-5-thiocyanobenzoic acid (NTCB), 1-cyano-4-dimethylaminopyridinium salt (DMAP-CN), and CN⁻ ion. The amount of said S-cyanylation reagent may be about 2 times to 50 times, more preferably about 5 times to 10 times, the total amount of all thiol groups.

Reaction temperature may be between about 0°C and 80°C, preferably between about 0°C and 50°C. The solvent used may be any buffer solution, as long as it does not react with any S-cyanylation reagent, and is exemplified by Tris-HCl buffer solution, Tris-acetate buffer solution, phosphate buffer solution, and borate buffer solution. Any organic solvent may be present, as long as it does not react with any S-cyanylation reagent.

It is recommended that said reaction be carried out at pH between 1 and 12. The pH is preferably between 7 and 10 when NTCB is used, and preferably between 2 and 7, for prevention of an S-S exchange reaction, when DMAP-CN is used. In the reaction mixture, a denaturant, such as guanidine hydrochloride, may be present.

The hydrolysis reaction described above is achieved by, for example, alkali treatment.

Said alkali treatment is carried out by adjusting the pH of an aqueous solution containing the starting compound to 7 to 14.

Said pH adjustment is normally achieved by adding an appropriate amount of a solution of sodium hydroxide, ammonia, an amino compound, the trisma base (tris[hydroxymethyl]-aminomethane), secondary sodium phosphate, potassium hydroxide, barium hydroxide, or the like, to an aqueous solution containing the starting compound, with preference given to sodium hydroxide etc.

The concentration of the solution used for the reaction described above is normally about 0.01 to 2 N, preferably about 0.05 to 1 N for sodium hydroxide, about 0.01 to 15 N, preferably about 0.1 to 3 N for ammonia or an amino compound, about 1 mM to 1 M, preferably about 20 mM to 200 mM for the trisma base, about 1 mM to 1 M, preferably about 10 mM to 100 mM for secondary sodium phosphate, and about 0.01 to 4 N, preferably about 0.1 to 2 N for potassium hydroxide. Reaction temperature may be any one between about -20°C to 80°C, more preferably between about -10°C and 50°C.

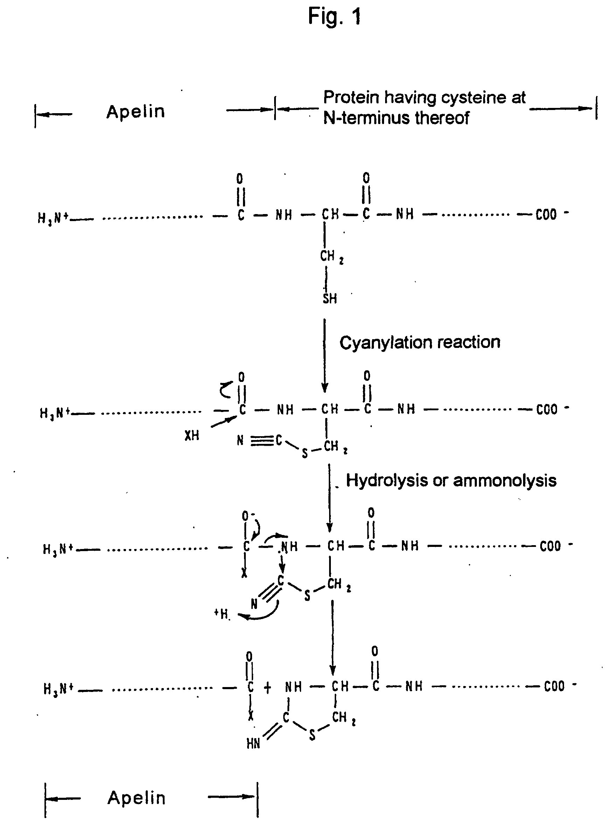

Reaction time is normally about 1 to 60 minutes, preferably about 15 to 30 minutes for an S-cyanylation reaction, about 5 minutes to 100 hours, preferably 10 minutes to 15 hours for a hydrolysis reaction, and about 5 minutes to 24 hours, preferably about 10 to 180 minutes for ammonolysis. It is hypothesized that upon the S-cyanylation or hydrolysis described above, the reaction shown by Fig. 1 takes place. With respect to Fig. 1, X represents R¹-(NR²)- (each of R¹ and R², whether identical or not, represents (i) hydrogen, (ii) C₁₋₂₀ alkyl, C₃₋₈ cycloalkyl, C₆₋₁₄ aryl or C₆₋₁₄ aryl-C₁₋₃ alkyl (these may have no substituent or may have 1 to 3 amino groups, hydroxyl groups, etc. on carbon atoms), (iii) an optionally substituted amino, (iv) a hydroxyl group or a C₁₋₆ alkoxy group) or OH.

Examples of such C₁₋₂₀ alkyl includes, for example, methyl, ethyl, propyl, isopropyl, butyl, sec-butyl, pentyl, isopentyl, neopentyl, 1-ethylpentyl, hexyl, isohexyl, heptyl, octyl, nonanyl, decanyl, undecanyl, dodecanyl, tetradecanyl, pentadecanyl, hexadecanyl, heptadecanyl, octadecanyl, nonadecanyl and eicosanyl.

Examples of such C₃₋₈ cycloalkyl includes, for example, cyclopropyl, cyclobutyl, cyclopentyl, cyclohexyl, cycloheptyl, and cyclooctyl.

Examples of such C₆₋₁₄ aryl includes phenyl, naphthyl, anthryl, phenanthryl, and acenaphthylenyl.

Examples of such C₆₋₁₄ aryl-C₁₋₃ alkyl includes, for example, benzyl, phenethyl, 3-phenylpropyl, (1naphthyl)methyl, and (2-naphthyl)methyl.

Examples of such C₁₋₆ alkoxy includes, for example, methoxy, ethoxy, propoxy, butoxy, pentyloxy, and hexyloxy.

Examples of substituents for such optionally substituted amino of (iii) above include, for example, amino acids and peptides consisting of 2 to 10 amino acids.

Such amino acids may be of the L-configuration or the D-configuration, and their examples include, for example, Ala, Arg, Asp, Asn, Glu, Gln, Gly, His, Ile, Met, Leu, Lys, Phe, Pro, Ser, Thr, Trp, Tyr, and Val.

Examples of such peptides include, for example, H-D-Leu-Leu-Arg-Pro-NH-C₂H₅ and H-Val-Ala-Leu-D-Ala-Ala-Pro-Leu-Ala-Pro-Arg-OH.

When ammonia or an amino compound is used in said reaction, a corresponding amide derivative is obtained.

The desired peptide cleaved out can be isolated using an ordinary method of peptide purification. For example, this isolation can be achieved by appropriately combining gel filtration, ion exchange chromatography, adsorption chromatography, high performance liquid chromatography, affinity chromatography, hydrophobicity chromatography, thin-layer chromatography, electrophoresis, etc. The thus-obtained desired peptide may have methionine of translation initiation codon origin added to the N-terminus thereof. It is also possible to remove the N-terminal methionine by reacting with an α-diketone, such as glyoxylic acid (preferably in the presence of a transition metal ion, such as copper sulfate ion, and a base, such as pyridine), and subsequently conducting hydrolysis using a diamine, such as o-phenylenediamine.

### [Method of removing N-terminal methionine of Apelin]

The term optionally oxidized methionine residue as used herein refers to a methionine residue or an S-oxide thereof, S-oxides of a methionine residue include sulfoxides and sulfone derivatives.

As Apelin having an optionally oxidized methionine residue at the N-terminus thereof, there may be mentioned peptides represented by the formula CH₃-S(O)ₘ-(CH₂)₂-CO-X [m represents an integer from 0 to 2; X represents a peptide chain of Apelin], which peptides may form salts; such salts are exemplified by the same as the salts of Apelin described above.

Apelin having an optionally oxidized methionine residue at the N-terminus thereof, or a salt thereof, is preferably Apelin having an optionally oxidized methionine residue at the N-terminus thereof, or a salt thereof, which is produced using gene engineering technology.

In the formula above, m is preferably 0. The peptide chain of Apelin represented by X is exemplified by the Apelin described above.

The Apelin or a salt thereof, described above, may undergo refolding (activation, reactivation) before or after being subjected to a removal process for the optionally oxidized methionine (Met) residue peptide at the N-terminus thereof or a diketone derivative of said methionine residue.

The term Apelin having a diketone derivative of an optionally oxidized methionine residue at the N-terminus thereof, or a salt thereof, refers to a compound represented by the formula CH₃-S(O)ₘ - (CH₂)₂-CO-CO-X [m represents an integer from 0 to 2; X represents a peptide chain of Apelin], or a salt thereof. Apelin having a diketone derivative of an optionally oxidized methionine residue at the N-terminus thereof, or a salt thereof, can be obtained by various reactions, such as chemical reactions or enzyme reactions. For example, by an amino group transamination reaction in which Apelin having an optionally oxidized methionine residue at the N-terminus thereof, or a salt thereof, is reacted with an α-diketone, Apelin having a diketone derivative of an optionally oxidized methionine residue at the N-terminus thereof, or a salt thereof, can be obtained.

In the present specification, any α-diketone is acceptable, as long as it is capable of proceeding the amino group transamination reaction of Apelin or a salt thereof described above, and is exemplified by a compound represented by the formula R¹-CO-CO-R² [R¹ represents hydrogen or a lower alkyl or phenyl group which may be substituted by a carboxyl group (preferably hydrogen or methyl, more preferably hydrogen); R² represents a hydroxyl group, a lower alkoxy group, or an amino group which may be substituted by a lower alkyl (preferably a hydroxyl group)] or a salt thereof.

The lower alkyl group represented by R¹ in the formula above is exemplified by a alkyl group having about 1 to 6 carbon atoms, such as methyl, ethyl, propyl, isopropyl, butyl, isobutyl, sec-butyl, and tert-butyl; the lower alkoxy group represented by R² is exemplified by an alkoxy group having 1 to 6 carbon atoms, such as methoxy, ethoxy, propoxy, iso-propoxy, butoxy, iso-butoxy, sec-butoxy, and tert-butoxy. In addition, an amino group which may be substituted by a lower alkyl represented by R² includes an amino group which may have 1 or 2 lower alkyl groups represented by R¹ above. Furthermore, as salts, there may be mentioned the same as the salts of Apelin having an optionally oxidized methionine residue at the N-terminus thereof described above.

Specific examples of α-diketone includes glyoxylic acid, pyruvic acid, oxalacetic acid, phenylglyoxylic acid, and 2-oxoglutaric acid, with preference given to glyoxylic acid.

The α-diketone may have formed a salt exemplified by alkali metal salts, such as sodium salt and potassium salt, and salts of inorganic acids, such as hydrochloride.

The amino group transamination reaction of Apelin having an optionally oxidized methionine residue at the N-terminus thereof or a salt thereof and an α-diketone is usually preferably carried out in a ratio of about 1 to 10,000 mol (preferably 2,000 to 4,000 mol) of α-diketone per mol of Apelin or a salt thereof at about 0 to 70°C (preferably about 20 to 40°C) for about 10 minutes to 5 hours (preferably about 20 minutes to 2 hours). Although any buffer solution (e.g., phosphate buffer solution, acetate buffer solution, citrate buffer solution) may be used, as long as the amino group transamination reaction described above is not interfered with, an acetate buffer solution is preferably used. It is recommended that the reaction be proceeded with the reaction pH adjusted to about 2 to 9, particularly about 4 to 7, and more particularly about 5 to 6.

To promote said amino group transamination reaction, it is preferable that an α-diketone be reacted in the presence of a transition metal ion; normally, it is preferable that a transition metal ion be used in a ratio of about 0.001 to 0.1 mol (preferably 0.01 to 0.05 mol) per mol of α-diketone. As transition metal ions, there may be used, for example, copper ions (Cu⁺, Cu²⁺), cobalt ions (Co²⁺, Co³⁺), nickel ions (Ni²⁺, Ni³⁺), iron ions (Fe²⁺, Fe³⁺), zinc ions (Zn²⁺), aluminum ions (Al³⁺), manganese ions (Mn²⁺ etc.), gallium ioins (Ga³⁺), indium ions (In³⁺), magnesium ions (Mg²⁺), and calcium ions (Ca²⁺), with preference given to copper ions, cobalt ions, etc., particularly copper ions (Cu²⁺). These transition metal ions can normally be added to the reaction solvent in the form of salts with inorganic acids, such as sulfuric acid, nitric acid, hydrochloric acid, and perchloric acid, or salts with organic acids, such as acetic acid, oxalic acid, citric acid, and carbonic acid, with preference given to copper sulfate and copper acetate, particularly copper sulfate.

It is also preferable that an α-diketone be reacted in the presence of a base; it is normally preferable that a base be used in a ratio of about 0.1 to 20 mol (preferably 0.5 to 10 mol) per mol of α-diketone. Useful bases include, for example, alkylamines, such as triethylamine and tributylamine, and aromatic amines, such as N,N-dimethylaniline, pyridine, lutidine, collidine, 4-(dimethylamino)pyridine, and imidazole, with preference given to pyridine.

In carrying out an amino group transamination reaction, it is preferable that urea be added to the buffer solution for the amino group transamination reaction for preventing the precipitation of Apelin having an optionally oxidized methionine residue at the N-terminus thereof or a salt thereof, and Apelin having a diketone derivative of a methionine residue obtained by the amino group transamination reaction of said Apelin or a salt thereof, or a salt thereof, and for other purposes. For example, it is preferable that urea be added to the buffer solution to a final concentration of about 1 to 8 M, more preferably about 3 to 6 M.

Furthermore, the amino group transamination reaction described above is preferably achieved by reacting an α-diketone in the presence of a transition metal ion and a base; practically, the amino group transamination reaction is proceeded in the presence of a mixture containing three components of a transition metal ion, a base, and an α-diketone (e.g., copper sulfate, pyridine, and glyoxylic acid), which mixture is added to an aqueous solution containing Apelin having an optionally oxidized methionine residue at the N-terminus thereof or a salt thereof.

Although the compound obtained by said amino group transamination reaction, represented by the formula CH₃-S(O)ₘ-(CH₂)₂-CO-CO-X [m represents an integer from 0 to 2; X represents a peptide chain of Apelin], or a salt thereof (Apelin having a diketone derivative of a methionine residue or a salt thereof) can be isolated and purified from the reaction solution by a known means of purifying peptides or proteins, e.g., extraction, salting-out, distribution, recrystallization, or chromatography, it may also be subjected to the next hydrolysis reaction as is.

The Apelin having a diketone derivative of methionine obtained by the amino group transamination reaction or a salt thereof can be converted to Apelin not having an optionally oxidized methionine residue at the N-terminal thereof, or not having a diketone derivative of said methionine residue or a salt thereof, normally by subjecting to hydrolysis with a base.

Bases used for the hydrolysis reaction include, for example, amines or salts thereof, including alkylamines, such as cysteamine, triethylamine, and tributylamine, or salts thereof; aromatic amines, such as N,N-dimethylaniline, pyridine, lutidine, collidine, 4-(dimethylamino)pyridine, and imidazole, or salts thereof; diamines, such as o-phenylenediamine, trylene-3,4-diamine, 3,4-diaminobenzoic acid and N-alkyl-substituted derivatives thereof (e.g., N-methyl-1,2-phenylenediamine, N-ethyl-1,2-phenylenediamine, N-isopropyl-1,2-phenylenediamine), 2,3-diaminophenol, and 4-chloro-o-phenylenediamine (preferable aromatic diamines, particularly 3,4-diaminobenzoic acid and N-alkyl-substituted derivatives thereof) (e.g., N-methyl-1,2-phenylenediamine, N-ethyl-1,2-phenylenediamine, N-isopropyl-1,2-phenylenediamine) or salts thereof; thiosemicarbazides, such as thiosemicarbazide, acetonethiosemicarbazide, and phenylthiosemicarbazide; and selenosemicarbazides, such as selenosemicarbazide and acetoneselenosemicarbazide; amines, particularly diamines or thiosemicarbazides or salts thereof are preferably used, with greater preference given to 3,4-diaminobenzoic acid or a salt thereof.

Salts of the base used for the hydrolysis reaction are exemplified by the same as the salts of Apelin having an optionally oxidized methionine residue at the N-terminus thereof described above.

The amount of the base is normally about 1 to 10,000 mol, preferably about 200 to 3,000 mol, and more preferably about 500 to 3,000 mol, per mol of Apelin having a diketone derivative of a methionine residue or a salt thereof. The hydrolysis reaction is normally preferably carried out at about 0 to 70°C (preferably about 20 to 40°C) for about 1 hour to 7 days (preferably about 10 hours to 5 days). The reaction is preferably carried out using a buffer solution as the solvent; such buffers include, for example, phosphate buffer solutions, acetate buffer solutions, citrate buffer solutions, and formate buffer solutions. Although any buffer solution may be used, as long as it does not interfere with the hydrolysis reaction described above, formate buffer solutions, such as acetate-sodium formate, formic acid-sodium formate or formic acid-sodium acetate buffer solution, are preferably used. It is recommended that the reaction be proceeded with the reaction pH adjusted to about 2 to 9, particularly about 3 to 7, and more particularly about 4 to 6. These buffer solutions are preferably used at about 0.1 to 8 mol/L, more preferably about 0.5 to 6 mol/L.

In conducting hydrolysis, it is preferable that urea be added to the buffer solution for the hydrolysis reaction for preventing the precipitation of Apelin having a diketone derivative of an optionally oxidized methionine residue at the N-terminus thereof or a salt thereof, and Apelin not having said methionine residue produced by the hydrolysis reaction or a salt thereof, and for other purposes. For example, it is preferable that urea be added to the buffer solution to a final concentration of about 1 to 6 M, more preferably about 2 to 5 M.

Although the thus obtained Apelin or a salt thereof can be isolated and purified from the reaction solution by a known means of purification, e.g., extraction, salting-out, distribution, recrystallization, or chromatography, examples of preferable means include, for example, ion exchange chromatography using SP-Sepharose (Pharmacia Biotech K.K.) or DEAE-5PW (Tosoh Corporation).

The Apelin obtained can be powdered by lyophilization as necessary. In carrying out lyophilization, a stabilizer, such as sorbitol, mannitol, dextrose, maltose, trehalose, or glycerol may be added.

The Apelin produced by the method of the present invention or a salt thereof can be mixed with sterile water, human serum albumin (HSA), physiological saline or another commonly known physiologically acceptable carrier, and can be administered non-orally or topically to mammals (e.g., humans). For example, it can be administered non-orally by intravenous injection, intramuscular injection, or the like, at a daily dose of about 0.01 mg to 50 mg, preferably about 0.1 mg to 10 mg, per human.

A preparation containing the Apelin produced using the method of the present invention or a salt thereof may contain other physiologically acceptable active components, such as salts, diluents, adjuvants, other carriers, buffers, binders, surfactants, and preservatives. A preparation for non-oral administration is supplied in ampoules of a suspension in a sterile aqueous solution or a physiologically acceptable solvent, or in ampoules of a sterile powder (normally obtained by lyophilizing a peptide solution) usable after being diluted freshly before use.

Because the Apelin obtained using the production method of the present invention is involved in central nervous function regulation, circulatory function regulation, immune function regulation, gastrointestinal function regulation, metabolic function regulation, reproductive function regulation, and the like, it can be used as a therapeutic or preventive agent for such diseases as various forms of dementia, including senile dementia, cerebrovascular dementia, dementia caused by systemic degeneration type retroplastic degenerative diseases (e.g., Alzheimer's disease, Parkinson's disease, Pick's disease, Huntington's disease), dementia caused by infectious diseases (e.g., slow virus infections such as Creutzfeldt-Jakob disease), dementia caused by endocrine, metabolic, or intoxication diseases (e.g., hypothyroidism, vitamin B12 deficiency, alcoholism, poisoning with various chemicals, metals, or organic compounds), and dementia caused by tumoral diseases (e.g., brain tumor), and dementia caused by traumatic diseases (e.g., chronic subdural hematoma), depression, hyperkinetic (minimal brain damage) syndrome, disturbance of consciousness, anxiety disorder, schizophrenia, phobia, impairments of growth hormone secretion (e.g., gigantism, acromegaly), hyperphagia, polyphagia, hypercholesterolemia, hyperglyceridemia, hyperlipidemia, hyperprolactinemia, hypoglycemia, hypopituitarism, pituitary dwarfism, diabetes mellitus (e.g., diabetic complications, diabetic nephropathy, diabetic neuropathy, diabetic retinopathy), cancers (e.g., breast cancer, lymphatic leukemia, lung cancer, bladder cancer, ovarian cancer, prostatic cancer), pancreatitis, renal diseases (e.g., chronic renal insufficiency, nephritis), Turner's syndrome, neurosis, rheumatoid arthritis, spinal injury, transient cerebral ischemic attack, amyotrophic lateral sclerosis, acute myocardial infarction, spinocerebellar deformation, bone fractures, wounds, atopic dermatitis, osteoporosis, asthma, epilepsy, infertility, arteriosclerosis, pulmonary emphysema, pulmonary edema, and milk secretion insufficiency. It can also be used as a hypnotic sedative, a postoperative nutritional status improving agent, a hypertensive, a hypotensive, or the like.

In addition, it can be used as a therapeutic or preventive agent for HIV infection, AIDS (acquired immune deficiency syndrome), etc., and the like.

Abbreviations for amino acids, peptides, protecting groups, activating groups, and others used in the present specification and attached drawings are based on abbreviations specified by the IUPAC-IUB Commission on Biochemical Nomenclature or abbreviations in common use in relevant fields. Some examples are given below. When an optical isomer may be present in amino acid etc., it is of the L-configuration, unless otherwise stated.
- DNA :: Deoxyribonucleic acid
- A :: Adenine
- T :: Thymine
- G :: Guanine
- C :: Cytosine
- RNA :: Ribonucleic acid
- EDTA :: Ethylenediaminetetraacetic acid
- Gly :: Glycine
- Ala :: Alanine
- Val :: Valine
- Leu :: Leucine
- Ile :: Isoleucine
- Ser :: Serine
- Thr :: Threonine
- Met :: Methionine
- Glu :: Glutamic acid
- Asp :: Aspartic acid
- Lys :: Lysine

- Arg :: Arginine
- His :: Histidine
- Phe :: Phenylalanine
- Tyr :: Tyrosine
- Trp :: Tryptophan
- Pro :: Proline
- Asn :: Asparagine
- Gln :: Glutamine
- Cys :: Cysteine
- ATP :: Adenosine triphosphate

The sequence numbers in the sequence listing in the present specification show the following respective sequences.
[SEQ ID NO: 1] Shows the amino acid sequence of Apelin-36.
[SEQ ID NO: 2] Shows the base sequence of the gene that encodes Apelin-36.
[SEQ ID NO: 3] Shows the amino acid sequence of bFGF CS23 mutein.
[SEQ ID NO: 4] Shows the base sequence of a fragment of the gene that encodes a fused protein represented by formula (I).
[SEQ ID NO: 5] Shows the base sequence of a fragment of the gene that encodes a fused protein represented by formula (I).
[SEQ ID NO: 6] Shows the base sequence of the gene that encodes a fragment of hbFGF mutein CS23.
[SEQ ID NO: 7] Shows the base sequence of a primer used to prepare the structural gene of Apelin-36 in Example 1.
[SEQ ID NO: 8] Shows the base sequence of a primer used to prepare the structural gene of Apelin-36 in Example 1.
[SEQ ID NO: 9] Shows the base sequence of a primer used to prepare the structural gene of Apelin-36 in Example 1.
[SEQ ID NO: 10] Shows the base sequence of a primer used to prepare the structural gene of Apelin-36 in Example 1.
[SEQ ID NO: 11] Shows the base sequence of a primer used to prepare the structural gene of Apelin-36 in Example 1.
[SEQ ID NO: 12] Shows the base sequence of a primer used to prepare the structural gene of Apelin-36 in Example 1.

### Examples

The present invention is hereinafter described in more detail by means of, but is not limited to, the following examples.

### Example 1: Preparation of structural gene for human Apelin-36

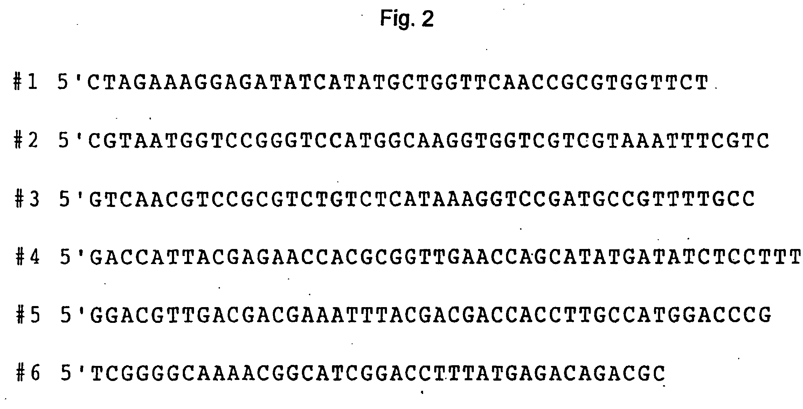

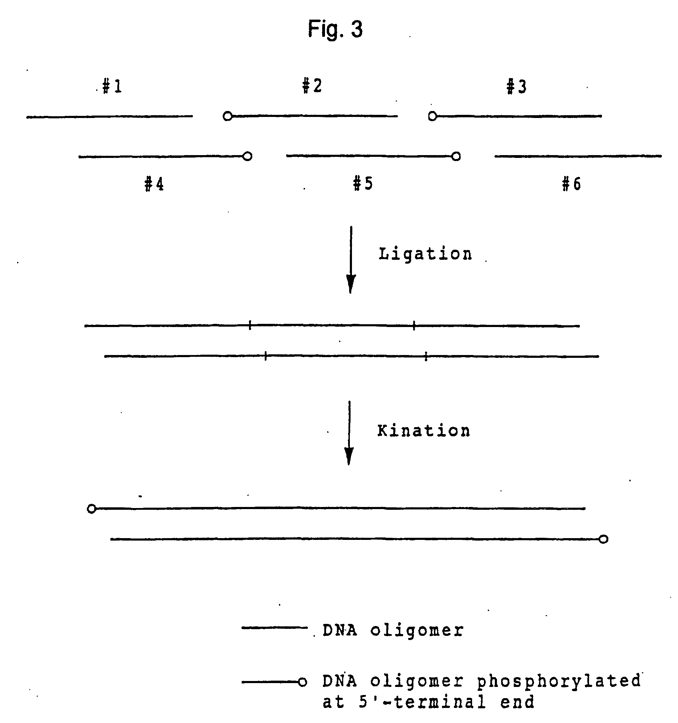

Using the six kinds of DNA fragments shown in Fig. 2 (#1 and #5: GRINER JAPAN, #2 and #6: KIKOTECH Co., Ltd., #3 and #4: Amersham-Pharmacia Biotech), the structural gene for Apelin-36 was prepared (Fig. 3).

### a) Phosphorylation of DNA oligomers

Four kinds of oligomers to serve as the 5'-terminus, 1 µg for each, was reacted at 37°C for 1 hour in 100 µL of a phosphorylation reaction mixture [50 mM Tris-HCl (pH 7.6), 10 mM MgCl₂, 1 mM spermidine, 10 mM dithiothreitol, 0.1 mg/mL bovine serum albumin, 1 mM ATP, 10 units of T4 polynucleotide kinase (Nippon Gene)] to phosphorylate the 5'-terminus. After phenol treatment, the water layer was recovered and ethanol was added in an amount doubling the water layer. After cooling to -70°C, DNA was precipitated by centrifugation.

### b) Ligation of DNA fragment

The phosphorylated DNA fragment obtained in a) above was combined with 1 µg of each of #1 and #2 to make a volume of 120 µL. This mixture was kept at 80°C for 10 minutes, after which it was cooled gradually to room temperature for annealing. A ligation reaction was carried out using the TaKaRa DNA Ligation Kit ver. 2 (Takara Shuzo). To 30 µL of the annealed liquid, 30 µL of liquid II was added. After the mixture was stirred vigorously, 60 µL of liquid I was added, followed by a ligation reaction at 37°C for 1 hour. After phenol treatment, the water layer was recovered and ethanol was added in an amount doubling the water layer. After cooling to -70°C, DNA was precipitated by centrifugation.

### c) Phosphorylation of the 5'-terminus

The precipitate was dissolved in 10 µL of a TE buffer solution (10 mM Tris-HCl (pH 8.0), 1 mM EDTA) and reacted at 37°C for 1 hour in 100 µL of a phosphorylation reaction mixture [50 mM Tris-HCl (pH 7.6), 10 mM MgCl₂, 1 mM spermidine, 10 mM dithiothreitol, 0.1 mg/mL bovine serum albumin, 1 mM ATP, 10 units of T4 polynucleotide kinase (Nippon Gene)] to phosphorylate the 5'-terminus. After phenol treatment, the water layer was recovered and ethanol was added in an amount doubling the water layer. After cooling to -70°C, DNA was precipitated by centrifugation and dissolved in 20 µL of the TE buffer solution.

### Example 2: Preparation of plasmid for human Apelin-36 expression

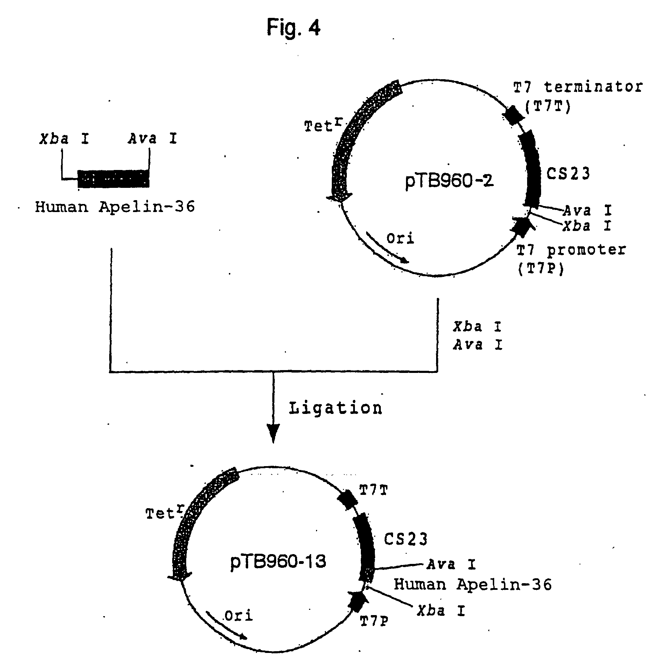

pTB960-2 (EP-A-499990: Koyama et al., Journal of Biotechnology, Vol. 32, p. 273, 1994) was digested with XbaI and AvaI and subjected to electrophoresis on 1% agarose; an about 4.4 Kbp DNA fragment was extracted using the QIAquick Gel Extraction Kit (Qiagen Company) and dissolved in 25 µL of a TE buffer solution. Using this XbI-AvaI fragment of pTB960-2 and the structural gene for human Apelin-36 as prepared above, a ligation reaction was carried out using the TaKaRa DNA Ligation Kit ver. 2 (Takara Shuzo). Specifically, 1 µL of the solution of the XbaI-AvaI fragment of pTB960-2 and 4 µL of a solution of the structural gene for human Apelin-36 were mixed. After 5 µL of liquid I was added to the mixture, a ligation reaction was carried out at 16°C for 30 minutes. Using 10 µL of the ligation mixture, *E*. *coli* JM109 competent cells (Toyobo) were transformed; the resulting transformant cells were sown over an LB-agar medium containing 10 µg/mL tetracycline and cultured at 37°C for 1 day; the resulting tetracycline-resistant colony was selected. This transformant was cultured overnight in LB medium, and the plasmid pTB960-13 was prepared using the QIAprep8 Miniprep Kit (QIAGEN Company). The base sequence of the portion of this plasmid corresponding to the structural gene for human Apelin-36 was determined using the Applied Biosystems model 377 DNA sequencer. The plasmid pTB960-13 was transformed to *E*. *coli* BL21 (DE3) strain (Novagen); the resulting transformant cells were sown over an LB-agar medium containing 10 µg/mL tetracycline and cultured at 37°C for 1 day to yield the human Apelin-36-CS23 fused protein-expressing strain BL21 (DE3)/pTB960-13 (Fig. 4). This transformant *E*. *coil* BL21 (DE3)/pTB960-13 was deposited under accession number FERM-BP6590 at the National Institute of Bioscience and Human-Technology, Agency of Industrial Science and Technology, Ministry of International Trade and Industry on December 2, 1998, and under accession number IFO16220 at the Institute for Fermentation (IFO) on November 11, 1998.

### Example 3

The transformant cells obtained in Example 2 were subjected to shaking culture at 37°C for 8 hours in a 2-liter flask using 1 liter of an LB medium containing 5.0 mg/L tetracycline (1% peptone, 0.5% yeast extract, 0.5% sodium chloride). The culture broth obtained was transferred to a 50-liter fermentation tank, previously charged with 19 liters of a main fermentation medium (1.68% sodium monohydrogen phosphate, 0.3% potassium dihydrogen phosphate, 0.1% ammonium chloride, 0.05% sodium chloride, 0.05% magnesium sulfate, 0.02% defoaming agent, 0.00025% ferrous sulfate, 0.00025% thiamine hydrochloride, 1.5% glucose, 1.5% casamino acid) and subjected to aerated spinner culture at 30°C. When the culture broth turbidity became approximately 500 Klett-value, isopropyl-β-D-thiogalactopyranoside was added to a final concentration of 12 mg/L, and cultivation was continued for 4 hours. After completion of the cultivation, the culture broth was centrifuged; about 660 g of wet cells were obtained and stored under freezing at -80°C.

### Example 4: Obtainment of human Apelin-36

To 550 g of the cells obtained in Example 2, 1,500 ml of a solution of 10 mM EDTA and 1 mM (p-amidinophenyl)methanesulfonyl fluoride hydrochloride (pH 6.0) was added. After sonication (BRANSON SONIFIER MODEL 450), the solution was centrifuged (10,000 rpm, 60 min). The supernatant was pooled, whereas the precipitate was subjected to the same operation. The pooled supernatant was adjusted to pH 6.0 and passed through a column of AF-Heparin Toyopearl 650 M (30 mm ID x 500 mmL, Tosoh), previously equilibrated with 50 mM phosphate buffer solution (pH 6.0). After protein adsorption and column washing, elution was conducted on a step-by-step gradient from 0 to 100% B (B = 50 mM phosphate buffer solution + 2 M NaCl, pH 6.0) to yield 530 ml of a human Apelin-36-CS23 fused protein fraction.

While 0.1 M acetic acid was added, this eluate was concentrated using a Pericon mini-cassette (Millipore) to yield a 0.1 M acetic acid solution of the human Apelin-36-CS23 fused protein. After urea was added to this solution to a final concentration of 6 M, 35 mg of 1-cyano-4-dimethylaminopyridinium salt (DMAP-CN) was added, followed by a reaction at room temperature for 15 minutes. After completion of the reaction, the reaction mixture was passed through a column of Sephadex G-25 (46 mm ID x 600 mmL, Pharmacia), previously equilibrated with 10% acetic acid. Elution was conducted at a flow rate of 6 ml/min of the 10% acetic acid used for the equilibration, to yield an S-cyanogenated human Apelin-36-CS23 fused protein fraction. This eluate was concentrated and desalinized using a Pericon mini-cassette (Millipore) to yield a desalinized solution of the human Apelin-36-CS23 fused protein. After urea was added to this desalinized solution to a final concentration of 6 M, 1 N caustic soda was added to a final concentration of 0.06 N, followed by a reaction at 0°C for 15 minutes. After completion of the reaction, the reaction mixture was adjusted to pH 6.0 with acetic acid to yield human Apelin-36. This reaction mixture was passed through a column of SP-5PW (21.5 mm ID x 150 mmL, Tosoh), previously equilibrated with a 50 mM phosphate buffer solution (pH 6.5) containing 3 M urea. After protein adsorption and column washing, elution was conducted on a step-by-step gradient from 0 to 40% B (B = 50 mM phosphate buffer solution + 1 M NaCl + 3 M urea, pH 6.5) to yield a human Apelin-36 fraction. This human Apelin-36 fraction was further passed through a column of C4P-50 (21.5 mm ID × 300 mmL, Showa Denko), previously equilibrated with 0.1% trifluoroacetic acid (TFA). After protein adsorption and column washing, elution was conducted on a step-by-step gradient from 15 to 30% B (B = 80% acetonitrile/0.1% TFA). After being pooled, the human Apelin-36 fraction was lyophilized to yield lyophilized powder of human Apelin-36.

### a) Amino acid composition analysis

The amino acid composition was determined using an amino acid analyzer (Hitachi L-8500A Amino Acid Analyzer). The amino acid composition determined was identical to the amino acid composition deduced from the DNA base sequence of human Apelin-36 with methionine added to the N-terminus thereof (Table 1).

**Table 1**

| Amino Acid Composition Analysis | | |
|---|---|---|
| Amino acid | Number of residues per mol | Value expected from the base sequence of human Apelin-36 |
| Asx | 1.0 | 1 |
| Thr¹⁾ | 0 | 0 |
| Ser¹⁾ | 1.9 | 2 |
| Glx | 3.0 | 3 |
| Pro | 5.7 | 6 |
| Gly | 5.7 | 6 |
| Ala | 0 | 0 |
| Cys²⁾ | N.D. | 0 |
| Val | 1.0 | 1 |
| Met | 2.0 | 1 |
| Ile | 0 | 0 |
| Leu | 2.0 | 2 |
| Tyr | 0 | 0 |
| Phe | 1.9 | 2 |
| His | 1.0 | 1 |
| Lys | 1.8 | 2 |
| Arg | 7.3 | 8 |
| Trp | 0.9 | 1 |

Acid hydrolysis (hydrolyzed with 6 N hydrochloric acid-4% thioglycolic acid at 110°C for 24 or 48 hours)
1) Value extrapolated to 0-hour time.
2) Not detected.

### b) N-terminal amino acid sequence analysis

The N-terminal amino acid sequence was determined using a gas phase protein sequencer (Applied Biosystems model 477A). The N-terminal amino acid sequence determined was identical to the N-terminal amino acid sequence deduced from the DNA base sequence of human Apelin-36, except for the addition of methionine to the N-terminus thereof (Table 2).

**Table 2**

| N-terminal Amino Acid Sequence | | |
|---|---|---|
| Residue No. | PTH¹⁾-amino acid detected (pmol) | Amino acid deduced from the base sequence of human Apelin-36 |
| 1 | Met (526) | |
| 2 | Leu (648) | Leu |
| 3 | Val (513) | Val |
| 4 | Gln (437) | Gln |
| 5 | Pro (463) | Pro |
| 6 | Arg (216) | Arg |
| 7 | Gly (232) | Gly |
| 8 | Ser (129) | Ser |
| 9 | Arg (129) | Arg |
| 10 | Asn (142) | Asn |
| 11 | Gly (185) | Gly |
| 12 | Pro (219) | Pro |
| 13 | Gly (202) | Gly |
| 14 | Pro (188) | Pro |
| 15 | Trp ( 88) | Trp |
| 16 | Gln (116) | Gln |
| 17 | Gly (120) | Gly |
| 18 | Gly ( 72) | Gly |
| 19 | Arg ( 56) | Arg |
| 20 | Arg ( 40) | Arg |
| Analysis was conducted using 1 nmol sample. | | |

### 1) Phenylthiohydantoin

### c) C-terminal amino acid analysis

The C-terminal amino acid was determined using an amino acid analyzer (Hitachi L-8500A Amino Acid Analyzer) (Table 3).

**Table 3**

| C-terminal Amino Acid Analysis | | |
|---|---|---|
| Human Apelin-36 | C-terminal amino acid | Recovery rate (%) |
| | Phe | 38.6 |
| Gas phase hydrazinolysis method (100°C, 6 hours) | | |

On the basis of these results, the human Apelin-36 obtained in Example 4 was identified as a molecular species with methionine added to the N-terminus thereof (Met-human Apelin-36).

### Example 5 (Determination of biological activity)

The activity of the Met-human Apelin-36 obtained in Example 4 was determined using the method described in Example 6 for WO 99/33976 (cytosensor) and was shown to be equivalent to the activity of a synthetic product.

### Example 6 (Removal of the methionine residue at the N-terminus: 1)

After 4 mg of the Met-human Apelin-36 obtained in Example 4 was dissolved in 0.8 ml of a 3 M urea solution, a mixture of 0.05 ml of 80 mM copper sulfate, 0.046 g of glyoxylic acid, and 0.1 ml of pyridine was added, followed by a reaction at 25°C for 1 hour. After completion of the reaction, the reaction mixture was passed through a column of Sephadex G-25 (10 mm ID × 250 mmL), previously equilibrated with 2.5 M urea + 10 mM phosphate buffer solution (pH 5.5). Elution was conducted at a flow rate of 0.5 ml/min of the solution used for the equilibration, and an Met-human Apelin-36 diketone derivative fraction was pooled. Subsequently, an equal amount of a solution of 4 M acetic acid, 4 M sodium acetate, and 3 M urea was added to this fraction, after which o-phenylenediamine was added to a final concentration of 40 mM, followed by degassing, gaseous nitrogen sealing, and a reaction at 25°C for 5 days. After completion of the reaction, the reaction mixture was passed through a column of Sephadex G-25 (25 mm ID × 600 mmL), previously equilibrated with a 50 mM phosphate buffer solution (pH 6.0). Elution was conducted at a flow rate of 4 ml/min of the buffer solution used for the equilibration, and a fraction of human Apelin-36 with no methionine added to the N-terminus thereof was pooled. The pooled human Apelin-36 fraction was adjusted to pH 6.0 and adsorbed to CM-5PW (7.5 mm ID × 75 mmL, Tosoh Corporation), previously equilibrated with a 50 mM phosphate buffer solution + 0.3 M NaCl + 2.5 M urea (pH 5.0), after which elution was conducted at a flow rate of 0.8 ml/min on a step-by-step gradient from 0 to 100% B (B = 50 mM borate buffer solution + 0.3 M NaCl + 2.5 M urea, pH 9.0) for 40 minutes, and a human Apelin-36 fraction was pooled. This human Apelin-36 was adsorbed to C4P-50 (10 mm ID × 250 mmL, Showa Denko), previously equilibrated with 0,1% TFA, after which it was eluted at a flow rate of 2 ml/min on a step-by-step gradient from 15 to 30% B (B = 80% acetonitrile/0.1% TFA) for 40 minutes. After being pooled, the human Apelin-36 fraction was lyophilized to yield human Apelin-36.

### a) Amino acid composition analysis

The amino acid composition was determined using an amino acid analyzer (Hitachi L-8500A Amino Acid Analyzer). The amino acid composition determined was identical to the amino acid composition deduced from the DNA base sequence of human Apelin-36 (Table 4).

**Table 4**

| Amino Acid Composition Analysis | | |
|---|---|---|
| Amino acid | Number of residues per mol | Value expected from the base sequence of human Apelin-36 |
| Asx | 1.0 | 1 |
| Thr¹⁾ | 0 | 0 |
| Ser¹⁾ | 1.8 | 2 |
| Glx | 3.0 | 3 |
| Pro | 5.7 | 6 |
| Gly | 5.6 | 6 |
| Ala | 0 | 0 |
| Cys²⁾ | N.D. | 0 |
| Val | 1.0 | 1 |
| Met | 1.0 | 1 |
| Ile | 0 | 0 |
| Leu | 2.0 | 2 |
| Tyr | 0 | 0 |
| Phe | 1.8 | 2 |
| His | 1.0 | 1 |
| Lys | 1.8 | 2 |
| Arg | 7.2 | 8 |
| Trp | 0.9 | 1 |

Acid hydrolysis (hydrolyzed with 6 N hydrochloric acid-4% thioglycolic acid at 110°C for 24 or 48 hours)
1) Value extrapolated to 0-hour time.
2) Not detected.

### b) N-terminal amino acid sequence analysis

The N-terminal amino acid sequence was determined using a gas phase protein sequencer (Applied Biosystems model 477A). The N-terminal amino acid sequence determined was identical to the N-terminal amino acid sequence deduced from the DNA base sequence of the human Apelin-36 obtained (Table 5).

**Table 5**

| N-terminal Amino Acid Sequence | | |
|---|---|---|
| Residue No. | PTH¹⁾-amino acid detected (pmol) | Amino acid deduced from the base sequence of human Apelin-36 |
| 1 | Leu (570) | Leu |
| 2 | Val (611) | Val |
| 3 | Gin (594) | Gin |
| 4 | Pro (587) | Pro |
| 5 | Arg (332) | Arg |
| 6 | Gly (552) | Gly |
| 7 | Ser (255) | Ser |
| 8 | Arg (277) | Arg |
| 9 | Asn (345) | Asn |
| 10 | Gly (383) | Gly |
| 11 | Pro (383) | Pro |
| 12 | Gly (366) | Gly |
| 13 | Pro (318) | Pro |
| 14 | Trp (131) | Trp |
| 15 | Gin (210) | Gin |
| 16 | Gly (218) | Gly |
| 17 | Gly (281) | Gly |
| 18 | Arg (130) | Arg |
| 19 | Arg (190) | Arg |
| 20 | Lys (144) | Lys |
| Analysis was conducted using 1 nmol sample. | | |

### 1) Phenylthiohydantoin

### c) C-terminal amino acid analysis

The C-terminal amino acid was determined using an amino acid analyzer (Hitachi L-8500A Amino Acid Analyzer) (Table 6).

**Table 6**

| C-terminal Amino Acid Analysis | | |
|---|---|---|
| Human Apelin-36 | C-terminal amino acid | Recovery rate (%) |
| | Phe | 66.3 |
| Gas phase hydrazinolysis method (100°C, 6 hours) | | |

### Example 7 (Determination of biological activity)

The activity of the human Apelin-36 obtained in Example 6 was determined using the method described in Example 6 for WO 99/33976 (cytosensor) and was shown to be equivalent to the activity of a synthetic product.

### Example 8 (Removal of the methionine residue at the N-terminus: 2)

After 4 mg of the Met-human Apelin-36 obtained in Example 4 was dissolved in 0.8 ml of a 3 M urea solution, a mixture of 0.05 ml of 80 mM copper sulfate, 0.046 g of glyoxylic acid, and 0.1 ml of pyridine was added, followed by a reaction at 25°C for 1 hour. After completion of the reaction, the reaction mixture was passed through a column of Sephadex G-25 (10 mm ID x 250 mmL), previously equilibrated with 2.5 M urea + 10 mM phosphate buffer solution (pH 5.5). Elution was conducted at a flow rate of 0.5 ml/min of the solution used for the equilibration, and a fraction of human Apelin-36 with a diketone derivative of a methionine residue was pooled. Subsequently, an equal amount of a solution of 2 M sodium formate, 4 M acetic acid, and 3 M urea was added to this fraction, after which 3,4-diaminobenzoic acid was added to a final concentration of 40 mM, followed by a reaction at 30°C for 3 days. After completion of the reaction, the reaction mixture was passed through a column of Sephadex G-25 (25 mm ID × 600 mmL), previously equilibrated with a 50 mM phosphate buffer solution (pH 6.0). Elution was conducted at a flow rate of 4 ml/min of the buffer solution used for the equilibration, and a fraction of human Apelin-36 with no methionine at the N-terminus thereof was pooled. The pooled human Apelin-36 fraction was adjusted to pH 6.0 and adsorbed to CM-5PW (7.5 mm ID × 75 mmL, Tosoh Corporation), previously equilibrated with a 50 mM phosphate buffer solution + 0.3 M NaCl + 2.5 M urea (pH 5.0), after which elution was conducted at a flow rate of 0.8 ml/min on a step-by-step gradient from 0 to 100% B (B = 50 mM borate buffer solution + 0.3 M NaCl + 2.5 M urea, pH 9.0) for 40 minutes, and a human Apelin-36 fraction was pooled. This human Apelin-36 was adsorbed to C4P-50 (10 mm ID × 250 mmL, Showa Denko), previously equilibrated with 0.1% TFA, after which it was eluted at a flow rate of 2 ml/min on a step-by-step gradient from 15 to 30% B (B = 80% acetonitrile/0.1% TFA) for 40 minutes. After being pooled, the human Apelin-36 fraction was lyophilized to yield human Apelin-36.

### a) Amino acid composition analysis

The amino acid composition was determined using an amino acid analyzer (Hitachi L-8500A Amino Acid Analyzer). The amino acid composition determined was identical to the amino acid composition deduced from the DNA base sequence of human Apelin-36 (Table 7).

**Table 7**

| Amino Acid Composition Analysis | | |
|---|---|---|
| Amino acid | Number of residues per mol | Value expected from the base sequence of human Apelin-36 |
| Asx | 1.0 | 1 |
| Thr¹⁾ | 0 | 0 |
| Ser¹⁾ | 1.9 | 2 |
| Glx | 2.9 | 3 |
| Pro | 6.3 | 6 |
| Gly | 5.9 | 6 |
| Ala | 0 | 0 |
| Cys^{2}} | N.D. | 0 |
| Val | 1.0 | 1 |
| Met | 1.0 | 1 |
| Ile | 0 | 0 |
| Leu | 2.0 | 2 |
| Tyr | 0 | 0 |
| Phe | 1.9 | 2 |
| His | 1.0 | 1 |
| Lys | 1.9 | 2 |
| Arg | 7.6 | 8 |
| Trp | 0.9 | 1 |

Acid hydrolysis (hydrolyzed with 6 N hydrochloric acid-4% thioglycolic acid at 110°C for 24 or 48 hours)
1) Value extrapolated to 0-hour time.
2) Not detected.

### b) N-terminal amino acid sequence analysis

The N-terminal amino acid sequence was determined using a gas phase protein sequencer (Applied Biosystems model 477A). The N-terminal amino acid sequence determined was identical to the N-terminal amino acid sequence deduced from the DNA base sequence of the human Apelin-36 obtained (Table 8).

**Table 8**

| N-terminal Amino Acid Sequence | | |
|---|---|---|
| Residue No. | PTH¹⁾-amino acid detected (pmol) | Amino acid deduced from the base sequence of human Apelin-36 |
| 1 | Leu (475) | Leu |
| 2 | Val (845) | Val |
| 3 | Gln (365) | Gin |
| 4 | Pro (563) | Pro |
| 5 | Arg (425) | Arg |
| 6 | Gly (424) | Gly |
| 7 | Ser (139) | Ser |
| 8 | Arg (423) | Arg |
| 9 | Asn (245) | Asn |
| 10 | Gly (290) | Gly |
| 11 | Pro (197) | Pro |
| 12 | Gly (234) | Gly |
| 13 | Pro (197) | Pro |
| 14 | Trp (101) | Trp |
| 15 | Gin ( 76) | Gin |
| 16 | Gly ( 84) | Gly |
| 17 | Gly (130) | Gly |
| 18 | Arg ( 79) | Arg |
| 19 | Arg (116) | Arg |
| 20 | Lys ( 43) | Lys |
| Analysis was conducted using 1 nmol sample. | | |

### 1) Phenylthiohydantoin

### c) C-terminal amino acid analysis

The C-terminal amino acid was determined using an amino acid analyzer (Hitachi L-8500A Amino Acid Analyzer) (Table 9).

**Table 9**

| C-terminal Amino Acid Analysis | | |
|---|---|---|
| Human Apelin-36 | C-terminal amino acid | Recovery rate (%) |
| | Phe | 86.6 |
| Gas phase hydrazinolysis method (100°C, 6 hours) | | |

### Example 9 (Determination of biological activity)

The activity of the human Apelin-36 obtained in Example 8 was determined using the method described in Example 6 for WO 99/33976 (cytosensor) and was shown to be equivalent to the activity of a synthetic product of human Apelin-36.

### INDUSTRIAL APPLICABILITY

Using the production method of the present invention, it is possible to produce on an industrial scale in large amounts a peptide that can be used as a therapeutic/preventive agent for such diseases as various forms of dementia, including senile dementia, cerebrovascular dementia, dementia caused by systemic degeneration type retroplastic degenerative diseases (e.g., Alzheimer's disease, Parkinson's disease, Pick's disease, Huntington's disease), dementia caused by infectious diseases (e.g., slow virus infections such as Creutzfeldt-Jakob disease), dementia caused by endocrine, metabolic, or intoxication diseases (e.g., hypothyroidism, vitamin B12 deficiency, alcoholism, poisoning with various chemicals, metals, or organic compounds), and dementia caused by tumoral diseases (e.g., brain tumor), and dementia caused by traumatic diseases (e.g., chronic subdural hematoma), depression, hyperkinetic (minimal brain damage) syndrome, disturbance of consciousness, anxiety disorder, schizophrenia, phobia, impairments of growth hormone secretion (e.g., gigantism, acromegaly), hyperphagia, polyphagia, hypercholesterolemia, hyperglyceridemia, hyperlipidemia, hyperprolactinemia, hypoglycemia, hypopituitarism, pituitary dwarfism, diabetes mellitus (e.g., diabetic complications, diabetic nephropathy, diabetic neuropathy, diabetic retinopathy), cancers (e.g., breast cancer, lymphatic leukemia, lung cancer, bladder cancer, ovarian cancer, prostatic cancer), pancreatitis, renal diseases (e.g., chronic renal insufficiency, nephritis), Turner's syndrome, neurosis, rheumatoid arthritis, spinal injury, transient cerebral ischemic attack, amyotrophic lateral sclerosis, acute myocardial infarction, spinocerebellar deformation, bone fractures, wounds, atopic dermatitis, osteoporosis, asthma, epilepsy, infertility, arteriosclerosis, pulmonary emphysema, pulmonary edema, and milk secretion insufficiency, and can also be used as a hypnotic sedative, a postoperative nutritional status improving agent, a hypertensive, a hypotensive, a preventive or therapeutic drug for HIV infection, AIDS (acquired immune deficiency syndrome), etc., and the like.

## Claims

1. A method for producing Apelin or a salt thereof, comprising subjecting a fused protein or peptide consisting of a protein or peptide having N-terminal cysteine with Apelin optionally having an optionally oxidized methionine residue at the N-terminus thereof joined to the N-terminus of the protein or peptide to a cleavage reaction for the peptide bond on the amino group side of the cysteine residue.

2. A method for producing Apelin optionally having a methionine residue at the N-terminus thereof or a salt thereof, comprising culturing a transformant harboring a vector having a gene that encodes a fused protein or peptide consisting of a protein or peptide having N-terminal cysteine with Apelin optionally having a methionine residue at the N-terminus thereof joined to the N-terminus of the protein or peptide, to allow it to express the fused protein or peptide, and subjecting the fused protein or peptide thus expressed to a cleavage reaction for the peptide bond on the amino group side of the cysteine residue.

3. The production method as claimed in claim 1 or 2, wherein said cleavage reaction involves an S-cyanylation reaction and a subsequent hydrolysis reaction.

4. The production method as claimed in claim 1 or 2, wherein said Apelin is a polypeptide containing the amino acid sequence shown by SEQ ID NO: 1.

5. A fused protein or peptide consisting of a protein or peptide having N-terminal cysteine with Apelin optionally having a methionine residue at the N-terminus thereof joined to the N-terminus of the protein or peptide.

6. A vector having a gene that encodes a fused protein or peptide as claimed in claim 5.

7. A transformant containing a vector as claimed in claim 6.

8. A method for removing an optionally oxidized methionine residue, comprising reacting Apelin having the optionally oxidized methionine residue at the N-terminus thereof or a salt thereof with an α-diketone, and subsequently conducting hydrolysis.

9. The method as claimed in claim 8, wherein said Apelin having an optionally oxidized methionine residue at the N-terminus thereof is produced using gene engineering technology.

10. The method as claimed in claim 8, comprising reacting with an α-diketone in the presence of a transition metal ion.

11. The method as claimed in claim 8, comprising reacting with an α-diketone in the presence of a base.

12. The method as claimed in claim 8, comprising reacting with an α-diketone in the presence of a transition metal ion and a base.

13. The method as claimed in claim 8, wherein said α-diketone is glyoxylic acid or a salt thereof.

14. The method as claimed in claim 10, wherein said transition metal ion is a copper ion.

15. The method as claimed in claim 11, wherein said base is pyridine.

16. The method as claimed in claim 8, comprising conducting hydrolysis using a base.

17. The method as claimed in claim 16, wherein said base is an amine.

18. The method as claimed in claim 16, wherein said base is a diamine or a thio or selenosemicarbazide.

19. The method as claimed in claim 18, wherein said diamine is o-phenylenediamine or 3,4-diaminobenzoic acid.

20. A method for producing Apelin or a salt thereof, comprising reacting Apelin produced using gene engineering technology and having methionine added to the N-terminus thereof or a salt thereof with glyoxylic acid or a salt thereof in the presence of copper sulfate and pyridine, and subsequently reacting with o-phenylenediamine or 3,4-diaminobenzoic acid.

21. A compound represented by the formula CH₃-S(O)ₘ-(CH₂)₂-CO-CO-X [m represents an integer from 0 to 2; X represents a peptide chain of Apelin] or a salt thereof.

22. A method for producing Apelin or a salt thereof, comprising hydrolyzing a compound as claimed in claim 21.

23. A method for producing Apelin or a salt thereof, comprising subjecting a fused protein or peptide consisting of a protein or peptide having N-terminal cysteine with Apelin having a methionine residue at the N-terminus thereof joined to the N-terminus of the protein or peptide to a cleavage reaction for the peptide bond on the amino group side of the cysteine residue, to yield Apelin having an optionally oxidized methionine residue at the N-terminus thereof or a salt thereof, reacting said Apelin having an optionally oxidized methionine residue at the N-terminus thereof or a salt thereof with an α-diketone, and subsequently conducting hydrolysis.
